(19)

Europäisches Patentamt

European Patent Office

Office européen des brevets

(11) **EP 4 516 235 A1**

(12) **EUROPEAN PATENT APPLICATION**

(43) Date of publication:
**05.03.2025 Bulletin 2025/10**

(21) Application number: **23194147.7**

(22) Date of filing: **30.08.2023**

(51) International Patent Classification (IPC):
**A61B 6/42** (2024.01)   **A61B 6/00** (2024.01)
**G21K 1/02** (2006.01)

(52) Cooperative Patent Classification (CPC):
**A61B 6/4291; A61B 6/483; A61B 6/5282; G21K 1/025**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **SINGH, Nishant**
  **5656AG Eindhoven (NL)**
• **JACOBS, Johannes Wilhelmus Maria**
  **5656AG Eindhoven (NL)**

(74) Representative: **Philips Intellectual Property & Standards**
**High Tech Campus 52**
**5656 AG Eindhoven (NL)**

(54) **ANTISCATTER GRIDS FOR X-RAY IMAGING SYSTEMS**

(57)   Known antiscatter grids need to be physically removed from X-ray imaging system at undesirable times, which disrupts the radiology workflow. The present disclosure provides an antiscatter grid (100) for an X-ray imaging system, the antiscatter grid comprising a plurality of X-ray absorbing septa walls (102) separated by interspace material (104), wherein the septa walls are arranged to form an array of grid pixels having a grid ratio R, where $R = h / (p-w)$, where p is the grid pixel pitch, h is the septa wall height, and w is the septa wall thickness, wherein the septa walls have a septa area density $\rho A$, where $\rho A = n\,w$, where n is the septa material density, wherein the antiscatter grid has a center, and wherein the grid has a primary X-ray transmission $T_P$. The antiscatter grid is characterized in that: the grid ratio R in the center of the antiscatter grid is larger than or equal to 6; the septa area density $\rho A$ in the center of the antiscatter grid is larger than or equal to 0.04 g/cm$^2$; the primary X-ray transmission $T_P$ in the center of the antiscatter grid is larger than or equal to 80%; and the septa wall height h is less than or equal to 10 mm. This combination of design parameters enables the antiscatter grid to exhibit a quality improvement factor Q that is larger than one for a range of imaging applications, so that the grid may remain always on.

Fig. 1B

EP 4 516 235 A1

**Description**

FIELD OF THE INVENTION

The invention relates to an antiscatter grid for an X-ray imaging system, to an X-ray imaging system comprising the antiscatter grid, and to a method of operating the X-ray imaging system.

BACKGROUND OF THE INVENTION

Scattered X-ray radiation remains one of the major causes of image quality degradation in diagnostic and interventional medical X-ray imaging systems using flat panel detectors (FPDs). Antiscatter grids improve image quality by absorbing scattered radiation. However, different patients, clinical use cases etc. may have different requirements with respect to the imaging setup of the X-ray imaging systems. Therefore, in the prior art, the antiscatter grid is physically removed from the X-ray imaging system at undesirable times, which disrupts the workflow in the radiology room and interrupts clinical procedures.

SUMMARY OF THE INVENTION

It is an object of the invention to better address one or more of these concerns. The invention is defined by the independent claims. The dependent claims define advantageous embodiments.
A first aspect of invention provides an antiscatter grid for an X-ray imaging system, the antiscatter grid comprising a plurality of X-ray absorbing septa walls separated by interspace material, wherein the septa walls are arranged to form an array of grid pixels having a grid ratio R, where $R = h / (p-w)$, where p is the grid pixel pitch, h is the septa wall height, and w is the septa wall thickness, wherein the septa walls have a septa area density pA, where $pA = n\,w$, where n is the septa material density, wherein the antiscatter grid has a center, and wherein the grid has a primary X-ray transmission $T_P$; the antiscatter grid being characterized in that:

the grid ratio R in the center of the antiscatter grid is larger than or equal to 6; the septa area density pA in the center of the antiscatter grid is larger than or equal to 0.04 g/cm$^2$;
the primary X-ray transmission $T_P$ in the center of the antiscatter grid is larger than or equal to 80%; and
the septa wall height h is less than or equal to 10 mm.

In this way, a combination of design parameters is selected which permit the antiscatter grid advantageously to remain in the X-ray imaging system permanently. Relative to a system without grid, the antiscatter grid which exhibits these design parameters always improves image quality for a range of current clinical imaging applications. Consequently, there is no need to remove the grid from the X-ray imaging system, and the radiology workflow is rationalized. This ability to leave the antiscatter grid attached may be described as "grid always on".
In view of the fact that the antiscatter grid does not need to be removed between exposures, the antiscatter grid in one example may be irremovably attachable to the X-ray imaging system, for example to an X-ray detector of the X-ray imaging system.
As mentioned, the antiscatter grid in one example exhibits a quality improvement factor Q that is larger than one for a range of imaging applications. The said range of imaging applications may comprise one or more of: 2D projection imaging of thin objects; and an interventional procedure, for example a C-arm interventional procedure. By "thin objects" is meant, in one example, objects having a thickness of 5 cm or less.
In addition to the above-mentioned parameters, the antiscatter grid may exhibit a grid pixel pitch p of more than or equal to 1 mm, and/or a septa wall thickness w of between 20 and 120 $\mu$m, which are further associated with the ability to provide a quality improvement factor Q of more than one for the range of imaging applications.
Various other advantageous implementation options exist for the components of the antiscatter grid. For example, the septa walls may comprise tungsten or a tungsten alloy, or may comprise an x-ray absorbing material containing lead, tantalum, bismuth, gold, molybdenum, cobalt, chromium, or copper. The interspace material may consist of air, for example, or may comprise aerogel, foam, carbon, paper, cotton fibre, aluminium, or glass. The grid pixels may exhibit a square or hexagonal cross-sectional shape.
The antiscatter grid may exhibit a suitable outer form. For example, the antiscatter grid may be flat, or it may be curved, i.e. it exhibits a curved outer surface, for cooperation with a curved X-ray detector.
The primary X-ray transmission may be determined using any appropriate technique. In one particular example comprising air-spaced square or hexagonal pixels, the primary X-ray transmission may be determined according to $T_P = ((p-w) / p)^2$.
According to a second aspect, there is provided an X-ray detector comprising a housing and the antiscatter grid of the first aspect. The antiscatter grid may be permanently integrated in the housing of the detector, to realise the "grid always on" feature disclosed herein.

According to a third aspect, there is provided an X-ray imaging system comprising an X-ray source, an X-ray detector, and the antiscatter grid of the first aspect. The antiscatter grid is positioned between the X-ray source and the X-ray detector. The antiscatter grid may be irremovably attachable to the X-ray imaging system to realise the "grid always on" feature. The antiscatter grid may or may not form part of the X-ray detector. The X-ray detector may comprise an X-ray sensitive pixel array in a detector housing. The X-ray sensitive pixel array may be directed towards the X-ray source.

One advantage of the X-ray imaging system of the third aspect is that no positional alignment is required between the grid pixels of the antiscatter grid and detector pixels of the X-ray detector. Thus, in one example of the X-ray imaging system, the grid pixels are positioned independently of the detector pixels, that is, there is no mutual positional dependency between the pixel positioning, such as in CT systems.

According to a fourth aspect, there is provided a method of operating the X-ray imaging system of the third aspect, the method comprising using the system to acquire X-ray images of one or more subjects using at least two different imaging applications without removing or replacing the antiscatter grid, i.e., with the "grid always on".

The indefinite article "a" or "an" does not exclude a plurality. In addition, the articles "a" and "an" as used herein should generally be construed to mean "one or more" unless specified otherwise or clear from the context to be directed to a singular form.

Unless specified otherwise, or clear from the context, the phrases "one or more of A, B and C", "at least one of A, B, and C", and "A, B and/or C" as used herein are intended to mean all possible permutations of one or more of the listed items. That is, the phrase "A and/or B" means (A), (B), or (A and B), while the phrase "A, B, and/or C" means (A), (B), (C), (A and B), (A and C), (B and C), or (A, B, and C).

The term "comprising" does not exclude other elements or steps. Furthermore, the terms "comprising", "including", "having" and the like may be used interchangeably herein.

The invention may include one or more aspects, examples or features in isolation or combination whether specifically disclosed in that combination or in isolation. Any optional feature or sub-aspect of one of the above aspects applies as appropriate to any of the other aspects.

These and other aspects will become apparent from, and elucidated with, reference to the detailed description provided hereinafter.

## BRIEF DESCRIPTION OF THE DRAWINGS

A detailed description will now be given, by way of example only, with reference to the accompanying drawings, in which:

FIG. 1A illustrates one example of a one-dimensional (1D) antiscatter grid;
FIG. 1B illustrates one example of a two-dimensional (2D) antiscatter grid;
FIGS. 2-4 are plots of Q-factor as function of water slab thickness for a number of 1D and 2D antiscatter grids exhibiting varying design parameters.

## DETAILED DESCRIPTION OF EMBODIMENTS

In current practice, FPD-based X-ray imaging systems are often used for many different clinical applications and patients. For example, diagnostic X-ray imaging systems are used for both radiography and fluoroscopy applications and interventional systems are used for both 2D and 3D imaging. Known antiscatter grids often need to be removed or replaced when a new patient is to be imaged, or even during a clinical procedure which involves different imaging applications. This reduces system utilisation and workflow efficiency and furthermore causes errors and disturbances, while reducing staff satisfaction and patient outcome.

The present disclosure proposes an antiscatter grid which can remain permanently attached to the system and does not need to be removed from the system ("grid always on"). This new design of antiscatter grid provides high performance over a broad range of imaging applications and exposure conditions, in particular in environments with low and high amounts of scatter, i.e. low and high scatter-to-primary ratio (SPR).

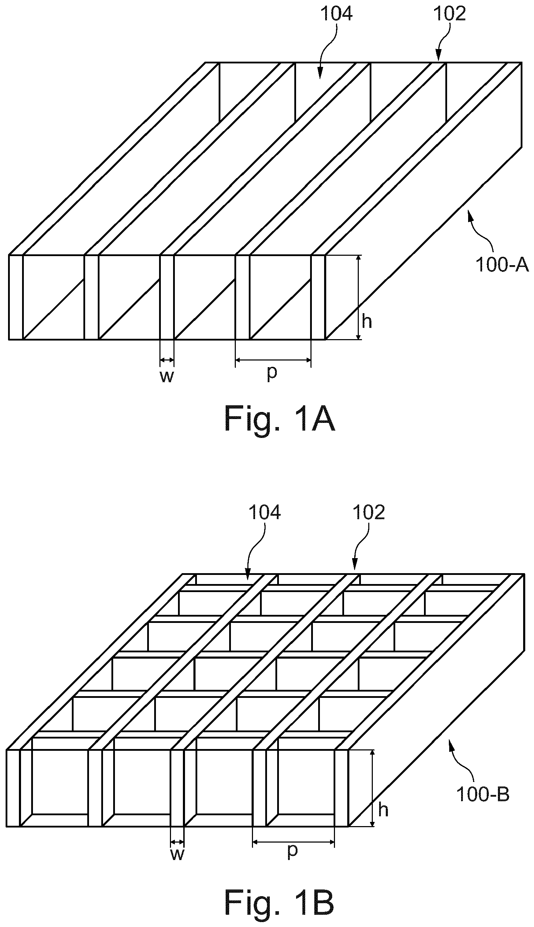

FIGS. 1A and 1B respectively illustrate an exemplary one-dimensional (1D) antiscatter grid 100-A and an exemplary two-dimensional (2D) antiscatter grid 100-B. Each antiscatter grid 100 comprises a plurality of X-ray absorbing septa walls 102 separated by interspace material 104. The septa walls 102 are arranged to form an array of grid pixels having a grid ratio R, where $R = h / (p-w)$, where p is the grid pixel pitch, h is the septa wall height, and w is the septa wall thickness. The septa walls 102 have a septa area density pA, where $pA = n\ w$, where n is the septa material density.

According to the present disclosure, certain design parameters for the antiscatter grid 100 are chosen to enable the antiscatter grid 100 to remain permanently present in the X-ray imaging system during all X-ray exposures, i.e., the antiscatter grid 100 does not need to be removed from the X- ray imaging system.

In one particular example, the antiscatter grid 100 exhibits the following characteristics:

the grid ratio R in the center of the antiscatter grid 100 is larger than or equal to 6;
the septa area density pA in the center of the antiscatter grid 100 is larger than or equal to 0.04 g/cm²;
the primary X-ray transmission $T_P$ in the center of the antiscatter grid 100 is larger than or equal to 80%; and
the septa wall height h is less than or equal to 10 mm.

There follows a discussion of two typical imaging applications in which the antiscatter grid 100 as described herein is able to improve image quality where the effect of a known antiscatter grid would be negative.

A first exemplary imaging application of a range of such imaging applications involves 2D projection imaging (e.g. radiographic or fluoroscopic examinations) of thin objects, which is characterized by a low scatter-to-primary ratio (SPR). This includes extremities for patients of all sizes and all types of views for small patients, including many pediatric patients. Pediatric patients are more radiosensitive than adults and therefore the dose is kept as low as practicable without compromising the diagnostic usefulness of the image. Hence, imaging small patients calls for a balance to be struck between image quality and patient dose. Removing known antiscatter grids from the X-ray imaging system reduces patient dose but also image contrast, meaning that the benefit of using the known antiscatter grids decreases with patient size, since the positive effect of scatter rejection becomes more and more outweighed by the negative effect of signal loss due to absorption of primary x-rays in the antiscatter grid. This deficit is overcome by the antiscatter grids described herein.

A second exemplary imaging application involves a C-arm interventional procedure involving a 3D cone beam CT (CBCT) image acquisition, in which the distance from the detector (and therefore from the grid) to the object is increased to enable more than 180 degrees of rotation of the C- arm around the patient. This reduces the SPR significantly and thereby the amount of scatter rejection by the antiscatter grid. Even in such scenarios, the antiscatter grids as described herein may improve image quality without needing to be removed.

Image quality may be quantified in any appropriate manner. For the purposes of the present disclosure, the grid quality improvement factor Q is the chosen metric for characterizing the benefit of the antiscatter grid for the application in any type of digital imaging. The grid quality improvement factor Q combines the contrast improvement ratio of an antiscatter grid with its primary radiation transmission $T_P$. More particularly, Q equals the square of the ratio of the signal-to-noise (SNR) with the grid and the SNR without the grid:

$$Q = ( \ SNR_{grid} \ / \ SNR_{no \ grid})^2 \quad (1)$$

which may be calculated as:

$$Q = ( \ T_{P, \ grid} \ / \ T_{P, \ no \ grid})^2 \ / \ (( \ T_{P, \ grid} + T_{S, \ grid} ) \ / \ ( \ T_{P, \ no \ grid} + T_{S, \ no \ grid} )) \qquad (2)$$

in which $T_{P, \ grid}$ and $T_{P, \ no \ grid}$ are the primary radiation transmission of the object captured by the detector in the presence and in the absence of the antiscatter grid, respectively, whereas $T_{S, \ grid}$ and $T_{S, \ no \ grid}$ are the scattered radiation transmission of the object captured by the detector in the presence and in the absence of the grid, respectively. In the present disclosure, $T_{P, \ grid}$ may be abbreviated as $T_P$ and $T_{S, \ grid}$ as $T_S$.

The grid quality improvement factor Q calculated by applying equation (2) is invariant under a scaling of the number of X-ray photons and is therefore independent of the radiation dose applied. The value of Q expresses to what extent a grid improves image quality by removing scattered X- ray photons while transmitting primary X-ray photons. Image quality is thus improved by the antiscatter grid when Q>1, image quality is not influenced when Q=1, and image quality is degraded when Q<1. Hence, the antiscatter grid may remain "always on" whenever Q>1.

The Q-factor depends on several factors including the scatter-to-primary ratio (SPR), which is determined primarily by properties and dimensions of the object (e.g. thickness), the geometry of the X-ray system (e.g. object-to-detector distance), and properties of the X-ray source (e.g. X-ray spectrum), and furthermore on the grid design, which is determined primarily by septa wall geometry and dimensions, septa and interspacer material properties, grid ratio, grid pixel pitch, and septa wall height.

The present disclosure is based on the recognition that the grid design parameters can be manipulated to ensure the condition that Q >1 for a broad range of object thicknesses and X-ray geometries which are commonly used in current practice of medical X-ray imaging. The antiscatter grids described herein ensure that in current medical imaging applications the critical condition Q>1 is fulfilled for at least imaging objects thicker than 5 cm, provided that a set of grid design rules is obeyed, meaning that there is no need to remove the antiscatter grid from the medical X-ray imaging system when the object and/or application is changed.

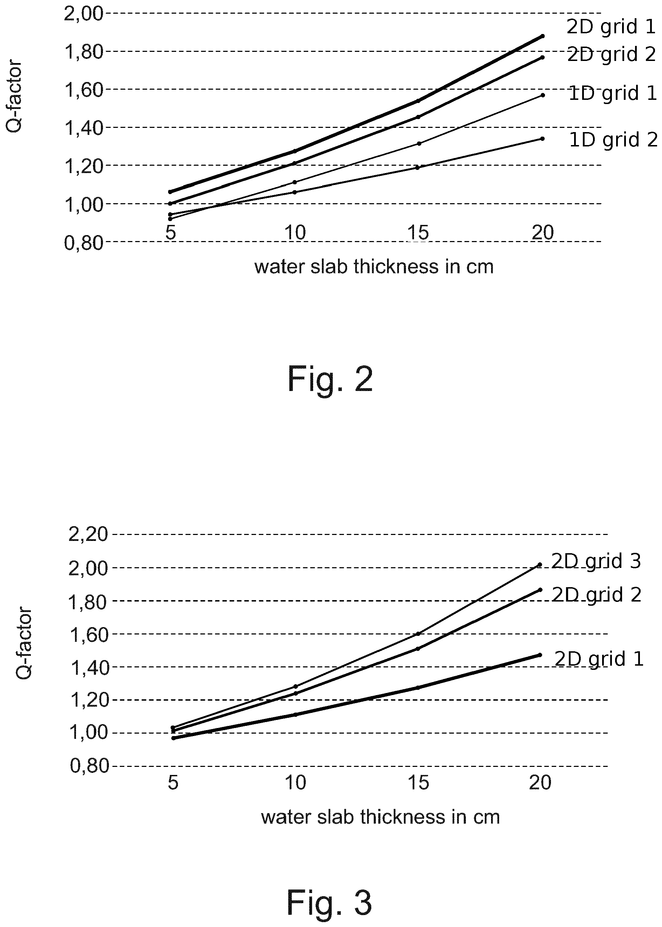

FIGS. 2-4 are plots of Q-factor as function of water slab thickness for a number of 1D and 2D antiscatter grids exhibiting varying design parameters. The Q-factor was determined by Monte Carlo (MC) simulations for various 1D and 2D grid designs using the following X-ray exposure conditions. The X-ray spectrum was emitted from a tungsten-anode based Philips X-ray tube SRM 0608 set at a certain peak voltage peak kVp without filtration of the X-ray beam. As phantom, a water slab of infinite lateral dimensions and a defined thickness between 5 and 25 cm was used. The X-ray geometry setup was fully symmetric with the X-ray tube and the phantom positioned above the center of the detector image plane at distances SID and DAD, respectively. The source-to-image distance (SID) is defined as the distance between the X-ray focal spot and the detector image plane. The detector-axis distance (DAD) is defined as the distance between the center of the water slab phantom and the detector image plane. A fixed distance gap of 2 cm was used between the detector-facing side of the grid and the detector image plane. Monte-Carlo simulations were carried in two successive steps. Firstly, an X-ray photon database was generated in a simulation tool by bombarding a large number (1-5 billion) of X-ray photons emitted from the X-ray focal spot of the tube on the water slab phantom. Secondly, another simulation tool took all relevant design parameters of a specific grid as input and then bombarded each photon from the generated photon database in the first step onto this grid. As a result, the numbers of primary and scatter transmission photons captured by a central area of 2x2 cm$^2$ on the detector was determined. This exercise was repeated for the case when no grid was present. The Q-factor was determined according to equation 2.

FIG. 2 illustrates calculated Q-factor as a function of water slab thickness for the following 1D and 2D antiscatter grids:

1D grid 1: standard Dunlee 1D grid (fiber interspacer, lead septa walls, R = 12, p = 227 $\mu$m, w = 36 $\mu$m and $T_P$ = 80%);
1D grid 2: 1D grid (air interspacer, tungsten septa walls, R = 5, p = 667 $\mu$m, w = 100 $\mu$m and $T_P$ = 85%);
2D grid 1: 2D grid (square pixels, air interspacer, tungsten septa walls, R = 5, p = 2 mm, w = 100 $\mu$m and $T_P$ = 90%);
2D grid 2: 2D grid (square pixels, air interspacer, tungsten septa walls, R = 5, p = 1.3 mm, w = 100 $\mu$m and $T_P$ = 85%).

For each of the four grids, the following parameters were chosen: X-ray tube voltage = 60 kVp, SID = 120 cm, DAD = 37 cm.

FIG. 2 shows that Q-factor increases with object thickness for both 1D grids and 2D grids. FIG. 2 also illustrates that, at equal primary X-ray transmission $T_P$, a 2D grid has a higher Q-factor than a 1D grid. In particular, FIG. 2 illustrates that the Q-factor of a specific 2D grid is higher than 1 for all objects thicker than 5 cm, when its primary X-ray transmission $T_P$ is 80% or more, or more particularly, 85% or more. For 2D grids with square or hexagonal grid pixels and air interspacer, it can be calculated geometrically that $T_P = ((p-w) / p)^2$, where p = grid pixel pitch and w = septa wall thickness. FIG. 3 illustrates Q-factor calculated as a function of water slab thickness for the following 2D antiscatter grids with increasing ratio R:

2D grid 1: R=3;
2D grid 2: R=6;
2D grid 3: R=12.

All three 2D grids investigated in relation to FIG. 3 have square pixels, air interspacer, tungsten septa walls, p =1.3 mm, w = 100 $\mu$m and $T_P$ = 85%. In each case, the following parameters were chosen: X-ray tube voltage = 60 kVp, SID = 120 cm, DAD = 37 cm.

FIG. 3 illustrates how the Q-factor of a 2D grid increases with increasing object thickness and increasing grid ratio R, which is defined as R = h / (p-w), in which p = grid pixel pitch, h = septa wall height and w = septa wall thickness. For all three 2D grids, pixel shape (square), interspacer material (air), X-ray exposure conditions, X-ray geometry, primary X-ray transmission ($T_P$ = 85%), grid pixel pitch (p = 1.3 mm), grid septa material (tungsten), and septa wall thickness (w = 100 $\mu$m) were kept constant. Only the grid ratio R was changed by adapting the septa wall height h. FIG. 3 illustrates that the Q-factor of a specific 2D grid is higher than 1 for all objects thicker than 5 cm, when its grid ratio R is 6 or more.

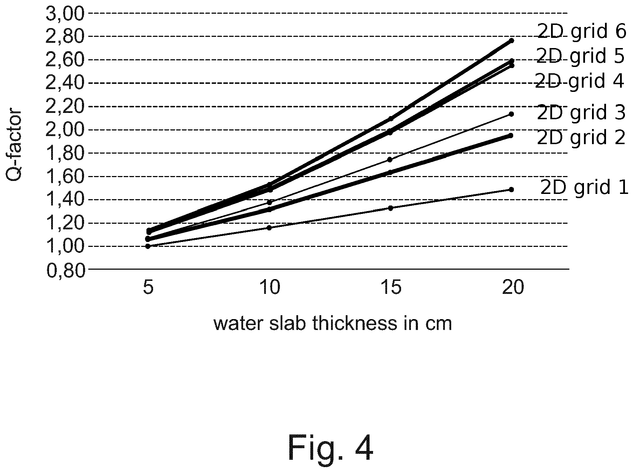

FIG. 4 illustrates Q-factor calculated as a function of water slab thickness for the following 2D grids with increasing $\rho_A$:

2D grid 1: $\rho_A$ = 0.02 g/cm$^2$, n = 10 g/cm$^3$, w = 20 $\mu$m, p = 0.3 mm;
2D grid 2: $\rho_A$ = 0.038 g/cm$^2$, n = 19 g/cm$^3$, w = 20 $\mu$m, p = 0.3 mm;
2D grid 3: $\rho_A$ = 0.05 g/cm$^2$, n = 10 g/cm$^3$, w = 50 $\mu$m, p = 0.75 mm;
2D grid 4: $\rho_A$ = 0.095 g/cm$^2$, n = 19 g/cm$^3$, w = 50 $\mu$m, p = 0.75 mm;
2D grid 5: $\rho_A$ = 0.1 g/cm$^2$, n = 10 g/cm$^3$, w = 100 $\mu$m, p = 1.5 mm;
2D grid 6: $\rho_A$ = 0.19 g/cm$^2$, n = 19 g/cm$^3$, w = 100 $\mu$m, p = 1.5 mm.

All six 2D grids have square pixels, air interspacer, tungsten septa walls, R = 5, and $T_P$ =87%, with the following parameters chosen: X-ray tube voltage = 120 kVp, SID = 90 cm, DAD = 22 cm.

FIG. 4 illustrates how Q-factor of a 2D grid increases with increasing object thickness and increasing septa area density pA, which is defined as pA = n w, in which n = septa material density, w = septa wall thickness. For all six simulated 2D grids, with square pixels and air interspacer, the X-ray exposure conditions, X-ray geometry, primary X-ray transmission ($T_P$ =85%), grid septa material (tungsten), and grid ratio (R = 5) were kept constant. Only the septa area density pA was varied by adapting the septa material density n, and/or the septa wall thickness w. FIG. 4 illustrates that the Q-factor of a specific 2D grid is higher than 1 for all objects thicker than 5 cm, when its septa area density pA is 0,038 g/cm$^2$ or more.

Further simulations not illustrated in the drawings indicate that, in clinical practice, the primary transmission $T_P$ in the corners of the 2D grid varies excessively when the SID is changed between different imaging applications, e.g. when switching from fluoroscopy to CBCT image acquisition, when the septa wall height h > 10 mm.

Optionally, the distance gap between the 2D grid and the detector may be chosen to reduce or minimize variation in the scatter profile. For some 2D grids (for example, those in which the grid pixel size is larger than the detector pixel size) there may be an optimal gap at which a desirably uniform (smooth) scatter profile is obtained. This is beneficial for the application of scatter correction methods. Further simulations indicate that the optimal gap, at which the scatter signal captured by the detector exhibits the least variation, increases with increasing 2D grid pixel pitch p. For example, the optimal gap is ca. 20 mm for p =1 mm, or 50 mm for p = 2.5 mm.

In some examples, the chosen set of 4 limit values in a multi-parameter space (R $\geq$ 6, pA $\geq$ 0.04 g/cm$^2$, Tp $\geq$ 80%, and h $\leq$ 10 mm) as described herein, based on the results of numerous MC simulations of different grid designs, may provide a degree of compromise or margin of safety regarding all possible practical grid designs. For example, all specific 2D grids in FIGS. 2-4 show that Q>1 when Tp $\geq$ 85%, but for most of these grids it would be possible to obtain Q > 1 for a chosen Tp between 80% and 85%, merely by varying other 2D grid design parameters.

The antiscatter grids described herein do not require accurate positional alignment between the 2D grid and the X-ray detector. This is in contrast with CT imaging systems in which detector pixels are mostly much larger (250-1000 $\mu$m) than in X-ray imaging systems (50-200 $\mu$m). In CT, the antiscatter grid and the detector are often positioned accurately relative to each other, so that grid septa walls overlap exactly with dead areas between detector pixels. This is done to maximize fill factor and thereby detector signal.

The antiscatter grids as described herein may be manufactured using fabrication methods such as additive manufacturing direct laser metal sintering (DMLS, 3D printing) or femto-laser based glass structuring.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. Measures recited in mutually different dependent claims may advantageously be combined. Any reference signs in the claims should not be construed as limiting the scope.

## Claims

1.    An antiscatter grid (100) for an X-ray imaging system, the antiscatter grid comprising a plurality of X-ray absorbing septa walls (102) separated by interspace material (104), wherein the septa walls are arranged to form an array of grid pixels having a grid ratio R, where R = h / (p-w), where p is the grid pixel pitch, h is the septa wall height, and w is the septa wall thickness, wherein the septa walls have a septa area density pA, where pA = n w, where n is the septa material density, wherein the antiscatter grid has a center, and wherein the grid has a primary X-ray transmission $T_P$; the antiscatter grid being **characterized in that**:

the grid ratio R in the center of the antiscatter grid is larger than or equal to 6;
the septa area density pA in the center of the antiscatter grid is larger than or equal to 0.04 g/cm$^2$;
the primary X-ray transmission $T_P$ in the center of the antiscatter grid is larger than or equal to 80%; and
the septa wall height h is less than or equal to 10 mm.

2.    The antiscatter grid of claim 1, wherein the antiscatter grid is irremovably attachable to an X-ray detector of the X-ray imaging system.

3.    The antiscatter grid of claim 1 or 2, wherein the antiscatter grid exhibits a quality improvement factor Q that is larger than one for a range of imaging applications.

4.    The antiscatter grid of claim 3, wherein the range of imaging applications comprises one or more of: 2D projection imaging of thin objects; an interventional procedure.

**5.** The antiscatter grid of any preceding claim, wherein the grid pixel pitch p is more than or equal to 1 mm.

**6.** The antiscatter grid of any preceding claim, wherein the septa wall thickness w is between 20 and 120 $\mu$m.

**7.** The antiscatter grid of any preceding claim, wherein the septa walls (102) comprise tungsten or a tungsten alloy.

**8.** The antiscatter grid of any preceding claim, wherein the septa walls (102) comprise an x-ray absorbing material containing lead, tantalum, bismuth, gold, molybdenum, cobalt, chromium, or copper.

**9.** The antiscatter grid of any preceding claim, wherein the interspace material (104) consists of air.

**10.** The antiscatter grid of any preceding claim, wherein the interspace material (104) comprises aerogel, foam, carbon, paper, cotton fibre, aluminium, or glass.

**11.** The antiscatter grid of any preceding claim, wherein the grid pixels exhibit a square or hexagonal cross-sectional shape.

**12.** An X-ray detector comprising a housing and the antiscatter grid (100) according to any preceding claim, wherein the antiscatter grid is permanently integrated in the housing of the detector.

**13.** An X-ray imaging system comprising an X-ray source, an X-ray detector, and the antiscatter grid (100) according to any of claims 1-11 positioned between the X-ray source and the X-ray detector.

**14.** The X-ray imaging system of claim 13, wherein the grid pixels are positioned independently of the detector pixels.

**15.** A method of operating the X-ray imaging system of claim 13, the method comprising using the system to acquire X-ray images of one or more subjects using at least two different imaging applications without removing or replacing the antiscatter grid.

Fig. 1A

Fig. 1B

Fig. 2

Fig. 3

Fig. 4

Europäisches
Patentamt

European
Patent Office

Office européen
des brevets

# EUROPEAN SEARCH REPORT

**Application Number**

EP 23 19 4147

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 6 795 529 B1 (BARNES GARY T [US] ET AL) 21 September 2004 (2004-09-21) * Table I * | 1-15 | INV. A61B6/42 A61B6/00 G21K1/02 |
| Y | US 2009/003530 A1 (VAN VROONHOVEN JOZEF CORNELIS [NL]) 1 January 2009 (2009-01-01) * paragraph [0006] * | 1-15 | |
| Y | US 2002/168052 A1 (CASTLEBERRY DONALD EARL [US]) 14 November 2002 (2002-11-14) * paragraph [0030] * | 1-15 | |
| Y | JP 5 059521 B2 (INST TECH PRECISION ELECT) 24 October 2012 (2012-10-24) * paragraph [0012] * | 1-15 | |
| Y | US 2017/206996 A1 (BECK THOMAS J [US]) 20 July 2017 (2017-07-20) * paragraph [0102] * | 1-15 | |
| Y | US 5 721 761 A (FERLIC DANIEL J [US] ET AL) 24 February 1998 (1998-02-24) * col. 3, line 37 * | 6 | TECHNICAL FIELDS SEARCHED (IPC) A61B H05G G21K |
| Y | US 2023/225685 A1 (ALTUNBAS CEM [US]) 20 July 2023 (2023-07-20) * paragraphs [0166], [0165] * | 1,5,15 | |

The present search report has been drawn up for all claims

| Place of search | Date of completion of the search | Examiner |
|---|---|---|
| Munich | 23 January 2024 | Anscombe, Marcel |

EPO FORM 1503 03.82 (P04C01)

## ANNEX TO THE EUROPEAN SEARCH REPORT
## ON EUROPEAN PATENT APPLICATION NO.

**EP 23 19 4147**

This annex lists the patent family members relating to the patent documents cited in the above-mentioned European search report.
The members are as contained in the European Patent Office EDP file on
The European Patent Office is in no way liable for these particulars which are merely given for the purpose of information.

**23-01-2024**

| Patent document cited in search report | | Publication date | Patent family member(s) | | Publication date |
|---|---|---|---|---|---|
| US 6795529 | B1 | 21-09-2004 | NONE | | |
| US 2009003530 | A1 | 01-01-2009 | CN | 101326591 A | 17-12-2008 |
| | | | EP | 1964132 A2 | 03-09-2008 |
| | | | US | 2009003530 A1 | 01-01-2009 |
| | | | WO | 2007069115 A2 | 21-06-2007 |
| US 2002168052 | A1 | 14-11-2002 | AT | E305168 T1 | 15-10-2005 |
| | | | DE | 60113492 T2 | 29-06-2006 |
| | | | EP | 1185986 A1 | 13-03-2002 |
| | | | JP | 4746245 B2 | 10-08-2011 |
| | | | JP | 2003529087 A | 30-09-2003 |
| | | | KR | 20020026433 A | 10-04-2002 |
| | | | US | 6438210 B1 | 20-08-2002 |
| | | | US | 2002168052 A1 | 14-11-2002 |
| | | | WO | 0173794 A1 | 04-10-2001 |
| JP 5059521 | B2 | 24-10-2012 | JP | 5059521 B2 | 24-10-2012 |
| | | | JP | 2009050654 A | 12-03-2009 |
| US 2017206996 | A1 | 20-07-2017 | US | 2017206996 A1 | 20-07-2017 |
| | | | WO | 2016014806 A1 | 28-01-2016 |
| US 5721761 | A | 24-02-1998 | NONE | | |
| US 2023225685 | A1 | 20-07-2023 | NONE | | |

EPO FORM P0459

For more details about this annex : see Official Journal of the European Patent Office, No. 12/82